# EUROPEAN PATENT APPLICATION

(11) **EP 2 309 000 A1**
(43) Date of publication of application: **13.04.2011**
(21) Application number: 09170011.2
(22) Date of filing: 11.09.2009
(51) Int. Cl.: C12Q 1/68

(54) **Ecotoxicological response indicators**

(71) Applicant: The European Union, represented by the European Commission, 1049 Brussels (BE)
(72) Inventor: Bopp, Stephanie, 21038, Arolo di Leggiuno (VA) (IT); Lettieri, Teresa, 21026, Gavirate (VA) (IT)
(74) Representative: Office Freylinger

(57) **Abstract**

The invention concerns a nucleic acid selected from SEQ ID NO:1-7, or a fragment thereof, its use as an hybridization probe, a composition for detection of gene using at least one of these probes, wherein said composition is preferably a microarray, as well as a method of detecting polycyclic aromatic hydrocarbon pollutants in water and a method of assessing toxicity of a polycyclic aromatic hydrocarbon compounds using the marine diatom Thalassiosira pseudonana.

## Description

### Technical field

The present invention pertains to the field of ecotoxicology and especially to the assessment of pollution e.g. by polycyclic aromatic hydrocarbon compounds using reliable indicators. In particular, the present invention relates to the evaluation of toxicological response of organisms to pollutants by providing appropriate biomarkers.

### Background Art

Gene expression is a sensitive indicator of how cells and organisms adapt to changes in the external environment. The measurements of gene expression levels upon exposure to a chemical can both provide information about the mechanism of action of toxicants and form a sort of "genetic signature" from the pattern of gene expression changes it elicits both in vitro (Burczynski et al. 2000; Waring et al. 2001) and in vivo (Hamadeh et al. 2002). The development of such gene expression signatures would allow fast screening of unknown or suspected toxicants on the basis of their similarity to known toxicants.

The possibility of analyzing the effect of chemicals and environmental stressors on a large number of genes in a single experiment has led to the development of the field of toxicogenomics. Proponents of toxicogenomics aim to apply both mRNA and protein expression technology to study chemical effects in biological systems (Afshari et al. 1999; Lovett 2000; Olden and Guthrie 2001).

One challenge facing ecotoxicology is to understand the mechanism of action of toxicants on living organisms (Snape et al. 2004). Such knowledge would help to develop predictive simulation models of toxic effects, to link molecular biomarkers with population level effects, and then to anticipate ecological risk assessment issues for new chemicals. Gene expression profiles represent the primary level of integration between environmental factors and the genome, providing the basis for protein synthesis, which ultimately guides the response of organisms to external changes.

Thus, the analysis of gene expression changes is a powerful tool both to diagnose major stressors in natural populations and to analyze the mechanisms of such stress responses.

The enormous potential that lies in the successful incorporation of genomic data into ecotoxicology faces several challenges. The major challenge is the difficult task of taking into account intrinsic sources of variability in gene expression levels due to different physiological states, age, sex, and genetic polymorphisms in natural populations (Jin et al. 2001; Oleksiak et al. 2002; Ranz et al. 2003; Townsend et al. 2003). Somewhat related is the additional problem of properly interpreting array data in the presence of such large intrinsic variations and then relating changes in gene expression to significant ecotoxicological parameters (i.e., at the population level) such as survival, growth, and reproduction. A second major limitation is the high cost associated with the technology itself. These costs render repeat measures very expensive, and thus often only limited experimental data are available.

The expression of certain genes can vary considerably even under tightly controlled experimental conditions. Fay et al. (2004) observed that around 400 genes were differentially expressed when analyzing nine different strains of *Saccharomyces cerevisiae.* To minimize the effect of genetic polymorphisms on gene expression levels, investigators usually determine the toxic properties of chemicals using inbred strains of mice and rats or well-characterized strains of yeast. In natural populations of non-model organisms, two approaches can be used to determine the "normal" gene expression patterns (Neumann and Galvez 2002). Pooling RNA samples from a large number of individuals in the control group will provide a measure of the mean gene expression response. This approach has the advantage of requiring a low number of microarrays, thereby reducing the overall cost of the experiment, but it does not provide any information about the distribution of responses in the natural population. The other, more expensive approach consists of measuring gene expression patterns for each individual in the control population. This approach makes it possible to obtain both the mean expression pattern and its distribution.

An additional problem is the limited availability of DNA/RNA arrays for non-model organisms. Even if several techniques are available to identify subsets of genes that respond to environmental stressors, the lack of whole-genome arrays does not allow use of the full potential of microarrays.
From this point of view, it is reassuring that the number of fully sequenced genomes is expanding very fast. For example, the recent sequencing of the diatom algae *Thalassiosira pseudonana* (Armbrust et al. 2004) has added to the list a very important organism for ecotoxicology studies.

In "Gene regulation in the marine diatom Thalassiosira pseudonana upon exposure to polycyclic aromatic hydrocarbons (PAHs)", Gene 396 (2007), 293-302, the inventors investigated the effects of three polycyclic aromatic hydrocarbons (PAHs, pyrene, fluoranthene, and benzo[a]pyrene), either as single compound or as mixture, at molecular level. Dose-response curves for growth inhibition were determined and four concentrations eliciting from "no effect" up to a severe growth inhibition were chosen for further investigation to detect alterations at gene expression level by Real-Time PCR. Among the eight selected genes, the studies showed that *lacsA* and *sil3* seem to be regulated by PAH exposure, giving some hints about PAH response. In fact, *lacsA,* which is involved in the fatty acid metabolism, was found to be strongly up-regulated by all single PAHs, as well as by the mixture, whereas *sil3,* involved in the formation of the silica shell, was repressed by a factor up to three even at low PAH concentrations not eliciting any growth inhibition.

Although the two genes *lacsA* and *sil3* could serve as biomarkers for monitoring the presence of PAHs, it would be desirable to find further/other indicators, which would allow for a more sensitive and more reliable evaluation of the presence of environmentally relevant concentrations of contaminants, such as PAHs.

### Technical problem

Hence, it is an object of the present invention to provide alternative biomarkers, which alone or preferably in combination would allow to reliably evaluate pollution.

It would further be desirable to propose a method, which would allow the fast and reliable screening for known relevant toxicants or even for unknown or suspected toxicants on the basis of their similarity to these known toxicants.

### General Description of the Invention

Therefore, the present invention provides nucleic acid molecules usable e.g. as probes for genes whose transcript levels are modulated in a sample during a metabolic response to a toxic compound, such as polycyclic aromatic hydrocarbon compounds (PAH), see Table 5 below. The invention thus provides for nucleic acids selected from SEQ ID NO:1-7, or any fragment thereof.

Among these probes, those of sequences SEQ ID NO:1 and 2 are indicative for genes *hsp20* and *proteas* whose transcript levels are up-regulated in a *T. pseudonana* sample during a metabolic response to a toxic PAH compound.

The invention further concerns probes for nucleic acid molecules whose transcript levels are down-regulated in a sample during a metabolic response to a toxic compound, such as *dhg, eif2ak3, aatp, mcllhc, tp8567,* i.e. probes corresponding to SEQ ID NO:3 to 7.

Up-regulation or down-regulation is generally at least 1.4 fold, more preferably at least 2 fold, most preferably at least 3 fold or even higher.

In a further aspect, the invention provides a composition for detection of gene(s), wherein the composition comprises at least one polynucleotide probe, wherein said at least one polynucleotide probe is selected from polynucleotide sequences SEQ ID NO:1-7.

In a preferred embodiment, the above-mentioned composition is a microarray comprising a support on which one or more of the polynucleotide probes of polynucleotide sequences SEQ ID NO:1-7 have been immobilized.

Preferably, the composition comprises one or more polynucleotide probes of polynucleotide sequences SEQ ID NO:1-4 and optionally further probes selected from SEQ ID NO:5-7. For example, a preferred composition, e.g. a microarray, comprises any one, two, three or preferably even all of the sequences SEQ ID NO:1-4 and if desirable any one, two or preferably even all of the sequences SEQ ID NO:5-7. A still further preferred, the composition is a microarray on which all of sequences SEQ ID NO:1-7 have been immobilized.

In a still further embodiment, the composition also comprises further polynucleotide probe(s), such as those of polynucleotide sequences SEQ ID NO:8 and/or SEQ ID NO:9.

The composition preferably further also comprises probes indicative of housekeeping genes to be used as an internal control, such as for example a polynucleotide probe of polynucleotide sequence SEQ ID NO:10 indicative of gene *gapdh* expression.

As a composition and especially a microarray may comprise a important number of probes, it is clear that further nucleic acid probes may be included either as a control or for the purpose of detecting expression of further genes, in relation to response to PAHs or to other ecotoxicologically relevant compounds.

In a further aspect, the invention concerns a method of detecting polycyclic aromatic hydrocarbon pollutants in water comprising the following steps:
- culturing *Thalassiosira pseudonana* cells in said water,
- obtaining nucleic acid from said culture by isolation,
- detecting level of gene expression using a composition as described above.

The method preferably further comprises the comparison with gene expression levels in the absence of pollutants and/or with gene expression levels in the presence of known quantities of pollutants to either qualitatively or quantitatively evaluate the presence of PAH compounds.

In a still further aspect, the invention provides a method for screening a test compound or molecule for a metabolic response in order to assess its toxicity to the organism. Hence, there is provided a method for assessing toxicity of a polycyclic aromatic hydrocarbon test compound comprising the following steps:
- culturing *Thalassiosira. pseudonana* cells in a medium comprising varying concentrations of said test compound,
- obtaining nucleic acid from said culture by isolation,
- detecting level of gene expression using a composition as described above.

The method preferably further comprises the comparison with gene expression levels in the absence of PAHs and with gene expression levels in the presence of known quantities of known PAHs to either qualitatively or quantitatively evaluate the toxicity of the PAH test compound.

Hence, the present invention provides methods for assessing PAH pollution in water samples, for screening PAH test compounds for potential toxicological responses and for screening a sample's toxicological response to a particular PAH test compound.

Briefly, these methods entail culturing a *T. pseudonana* sample in the presence of a test compound or obtaining a *T. pseudonana* sample suspected to exposure to a test compound susceptible to elicit a change in gene expression patterns comprising the modulated (up-regulated or down-regulated) expression of a plurality of sample nucleic acid molecules.

As mentioned, the nucleic acid probes are preferably arrayed on a substrate. Then, the arrayed nucleic acid molecules and sample nucleic acid molecules are combined under conditions effective to form hybridization complexes, which may be detected by methods well known in the art. Detection of higher or lower levels of such hybridization complexes compared with hybridization complexes derived from samples treated with a compound that is known not to induce a toxicological response correlates with a toxicological response to a test compound. Furthermore, the comparison of the detected response levels with previously measured levels in response to known concentrations of known toxic compounds allows to quantify the (equivalent) toxicant concentrations to which the sample has been exposed.

In the present application the terms used have their meaning generally known in the art. However in order to further clarify the terms used, some expressions are explicitly defined as follows.

"Nucleic acid molecule" refers to a nucleic acid, oligonucleotide, nucleotide, polynucleotide or any fragment thereof. It may be DNA or RNA of genomic or synthetic origin, double-stranded or single-stranded, and combined with carbohydrate, lipids, protein or other materials to perform a particular activity such as transformation or form a useful composition such as a peptide nucleic acid (PNA).

A "polynucleotide" refers to a chain of nucleotides. Preferably, the chain has from about 5 to 10,000 nucleotides, more preferably from about 50 to 3,500 nucleotides. "Oligonucleotide" is substantially equivalent to the terms amplimer, primer, oligomer, element, target, and probe and is preferably single stranded.

"Protein" refers to an amino acid sequence, oligopeptide, peptide, polypeptide or portions thereof whether naturally occurring or synthetic.

"Up-regulated" refers to a nucleic acid molecule whose levels increased in a treated sample compared with the nucleic acid molecule in an untreated sample.

"Down-regulated" refers to nucleic acid molecule whose levels decreased in a treated sample compared with the nucleic acid molecule in an untreated sample.

"Toxic compound", "toxicant", "toxic agent" or "pollutant" is any compound, molecule, or agent that elicits a biochemical, metabolic, and physiological response in an individual, such as i) DNA damage, ii) cell damage, iii) organ damage or cell death, or iv) clinical morbidity or mortality.

"Toxicological response" refers to a biochemical, metabolic, and physiological response in an individual or test system which has been exposed to a toxic compound, toxicant, toxic agent or pollutant.

"PAHs", "poly-aromatic hydrocarbons" or "polycyclic aromatic hydrocarbons" include compounds such as pyrene, fluoranthene, and benzo[a]pyrene (BaP), 3-methylcholanthrene, benzo[a]anthracene, 7,12-dimethyl-benz[a]anthracene, etc., as well as their corresponding metabolites, and the like. In a preferred embodiment the toxic compound is pyrene, fluoranthene, and/or benzo[a]pyrene, or one of their metabolites, or mixtures thereof.

A "housekeeping gene" is typically a constitutive gene that is transcribed at a relatively constant level across many or all known conditions. The housekeeping gene's products are typically needed for maintenance of the cell. It is generally assumed that their expression is unaffected by experimental conditions.

A "composition" as used herein refers to the largest meaning of the word, i.e. a combination of distinct parts or elements. As such, a composition according to the invention may thus be a mixture or an arrangement, especially an array, such as preferably a microarray.

The term "microarray" refers to an arrangement of hybridizable array elements. The array elements are arranged so that there are preferably at least one or more different array elements, more preferably at least 10 array elements, and most preferably at least 100 array elements, and even more preferably 10,000, per cm² substrate surface. Furthermore, the hybridization signal from each of the array elements is individually distinguishable.

When the nucleic acid molecules are employed as hybridizable array elements in a microarray, the array elements can be randomly distributed or preferably organized in an ordered fashion so that each element is present at a specified location on the substrate. Because the array elements in the latter case are at specified locations on the substrate, the hybridization patterns and intensities (which together create a unique expression profile) can be interpreted in terms of expression levels of particular genes and can be correlated with a toxicological response associated with a test compound or molecule.

"Hybridization" is the process of combining complementary, single-stranded nucleic acids into a single molecule. In general, the sequences of these nucleic acids will be entirely complementary (no mismatches) when aligned during hybridization. However, the hybridization may also occur when not all of the base pairs are correctly paired, i.e. there may be some mismatch between strands, e.g. due to natural genetic variations existing in natural populations, without substantially compromising hybridization. Such mismatches will however generally not amount to more than about 10%.

The nucleic acid molecule sequences of the Sequence Listing have been prepared by current, state-of-the-art, automated methods and, as such, may contain occasional sequencing errors and unidentified nucleotides. Nucleotide analogues can be incorporated into the nucleic acid molecules by methods well known in the art. The only requirement is that the incorporated nucleotide analogues must serve to base pair with sample nucleic acid molecules. For example, certain guanine nucleotides can be substituted with hypoxanthine which base pairs with cytosine residues. However, these base pairs are less stable than those between guanine and cytosine.

Alternatively, adenine nucleotides can be substituted with 2,6-diaminopurine, which can form stronger base pairs than those between adenine and thymidine. Additionally, the nucleic acid molecules can include nucleotides that have been derivatized chemically or enzymatically.

Typical modifications include derivatization with acyl, alkyl, aryl or amino groups.

The nucleic acid molecules can be immobilized on a substrate via chemical bonding.

Furthermore, the molecules do not have to be directly bound to the substrate, but rather can be bound to the substrate through a linker group. The linker groups are typically about 6 to 50 atoms long to provide exposure to the bound nucleic acid molecule. Preferred linker groups include ethylene glycol oligomers, diamines, diacids and the like. Reactive groups on the substrate surface react with one of the terminal portions of the linker to bind the linker to the substrate. The other terminal portion of the linker is then functionalized for binding the nucleic acid molecule.

Preferred substrates are any suitable rigid or semi-rigid support, including membranes, filters, chips, slides, wafers, fibers, magnetic or nonmagnetic beads, gels, tubing, plates, polymers, microparticles and capillaries. The substrate can have a variety of surface forms, such as wells, trenches, pins, channels and pores, to which the arrayed nucleic acid molecules are bound.

DNA or RNA can be isolated from the sample according to any of a number of methods well known to those of skill in the art. For example, methods of purification of nucleic acids are described in Laboratory Techniques in Biochemistry and Molecular Biology: Hybridization With Nucleic Acid Probes, Part I. Theory and Nucleic Acid Preparation, P. Tijssen, ed. Elsevier (1993).

In a preferred embodiment, total RNA is isolated using the TRIZOL total RNA isolation reagent (Life Technologies, Gaithersburg MD). When sample nucleic acid molecules are amplified it is desirable to amplify the sample nucleic acid molecules and maintain the relative abundances of the original sample, including low abundance transcripts. RNA can be amplified in vitro, in situ or in vivo (see e.g. Eberwine, US Patent No. 5,514,545).

It is also advantageous to include (further) controls within the sample to assure that amplification and labeling procedures do not change the true distribution of nucleic acid molecules in the sample. For this purpose, a sample may be complemented with an amount of control nucleic acid molecules predetermined to be detectable upon hybridization to its complementary arrayed nucleic acid molecule and the composition of nucleic acid molecules includes reference nucleic acid molecules which specifically hybridize with the control arrayed nucleic acid molecules. After hybridization and processing, the hybridization signals obtained should reflect accurately the amounts of control arrayed nucleic acid molecules added to the sample.

The sample nucleic acid molecules may be labeled with one or more labeling moieties to allow for detection of hybridized arrayed/sample nucleic acid molecule complexes. The labeling moieties can include compositions that can be detected by spectroscopic, photochemical, biochemical, bioelectronic, immunochemical, electrical, optical or chemical means. The labeling moieties include radioisotopes, such as ³²P, ³³P or ³⁵S, chemiluminescent compounds, labeled binding proteins, heavy metal atoms, spectroscopic markers, such as fluorescent markers and dyes, magnetic labels, linked enzymes, mass spectrometry tags, spin labels, electron transfer donors and acceptors, and the like. Preferred fluorescent markers include Cy3 and Cy5 fluorophores (Amersham Pharmacia Biotech, Piscataway NJ).

The nucleic acid molecule sequences of SEQ ID NOs: 1-7, those of SEQ ID NOs: 8-10, and fragments thereof can be used in various hybridization technologies for various purposes in a test system. Hybridization or PCR probes may be produced using oligolabeling, nick translation, end-labeling, or PCR amplification in the presence of the labeled nucleotide. A vector containing the nucleic acid sequence may be used to produce an mRNA probe in vitro by addition of an RNA polymerase and labeled nucleic acid molecules. These procedures may be conducted using commercially available kits such as those provided by Amersham Pharmacia Biotech.

The stringency of hybridization is determined by G+C content of the probe, salt concentration, and temperature. In particular, stringency can be increased by reducing the concentration of salt or raising the hybridization temperature. In solutions used for some membrane based hybridizations, addition of an organic solvent such as formamide allows the reaction to occur at a lower temperature. Hybridization can be performed at low stringency with buffers, such as 5 x SSC with 1% sodium dodecyl sulfate (SDS) at 60 °C, which permits the formation of a hybridization complex between nucleotide sequences that contain some mismatches. Subsequent washes are performed at higher stringency with buffers such as 0.2 x SSC with 0.1 % SDS at either 45 °C (medium stringency) or 68 °C (high stringency). At high stringency, hybridization complexes will remain stable only where the nucleic acid sequences are completely complementary. In some membrane-based hybridizations, preferably 35% or most preferably 50%, formamide can be added to the hybridization solution to reduce the temperature at which hybridization is performed, and background signals can be reduced by the use of other detergents such as Sarkosyl or Triton X-1 00 and a blocking agent.

Selection of components and conditions for hybridization are well known to those skilled in the art and are reviewed e.g. in Sambrook et al. (1989) Molecular Cloning. A Laboratory Manual, Cold Spring Harbor Press, Plainview NY.

### Description of Preferred Embodiments

### 1. Diatom strain and routine culture

*Thalassiosira pseudonana* (strain CCMP 1335) was obtained as axenic culture from the Provasoli-Guillard National Center for Culture of Marine Phytoplankton (CCMP, West Boothbay Harbour, Maine, USA). Diatoms were maintained at 6-8ºC under a diurnal light cycle of 13 h light and 11 h darkness. The culture medium was f/2-medium (Guillard, 1975) based on 3.2% artificial sea water (ASW, Sigma-Aldrich, Steinheim, Germany). *T. pseudonana* was cultured in 250 mL Erlenmeyer flasks at densities between 0.5x10⁶ - 5x10⁶ cells/mL. Doubling times under these conditions were ca. 24 h. Fresh cultures for maintenance were inoculated every seven days.

### 2. Exposure of T. pseudonana to PAHs

T. pseudonana was exposed to the Polycyclic Aromatic Hydrocarbon (PAH) Benzo[a]pyrene (BaP) to detect effects of this compound group on growth and on gene expression. Growth inhibition was investigated in 20 mL batch cultures, which were inoculated from seven day old cultures to a start cell density of 5x10⁵ cells/mL. After a pre-incubation period of 24 h, to avoid lag-phases during exposure, cell density at the start of the exposure was measured as described below. BaP was dissolved in methanol and added to the culture leading to a methanol concentration of 0.05 % in the culture medium. Methanol was investigated before to have no effect on the diatom growth up to the highest tested concentration of 0.5 %. Dosing of methanol (solvent control) and BaP solution was performed 30 min before the light cycle started. BaP was tested at a concentration range of 0.05-328 µg/L using a dilution series with dilution factor 3. After 24 h of exposure, cell densities were measured in order to determine growth inhibition with respect to the untreated control cultures using the growth rates. Then, diatom cultures were centrifuged at 1500 rcf for 10 min. The supernatant was removed and the cell pellet resuspended in 0.5 mL PBS buffer (Gibco, Invitrogen, Paisley, UK). The suspension was transferred to 1.5 mL tubes and centrifuged at 10,000 rcf for 10 min. Then, supernatants were removed and the remaining cell pellets were frozen at -80 ºC.

Only samples treated with 0.05, 0.45, 4.05, and 36.45 µg/L BaP were investigated using real-time PCR. For microarray analyses, only samples treated with BaP concentrations of 36.45 µg/L were used. In some cases also samples from exposure to lower concentrations (4.05 µg/L) were examined. Samples used for microarray analyses stemmed from 5 independent biological experiments, which were performed over a period of 2 years.

### 3. Detection of growth inhibition

Cell density was determined at the end of the exposure period transferring each 200 µL of diatom suspension to 96 well plates (Falcon, BD Biosciences, Erembodegem, Belgium) and measuring the absorption at 450 nm using a microplate spectrophotometer (Biorad, Hercules, CA, USA). (Correlation between absorption and cell density was investigated previously, R² = 0.9997.) Growth rates were calculated from cell densities and used to determine growth inhibition compared to the control. Growth inhibition curves were fitted using a nonlinear regression sigmoidal dose response curve model provided in the GraphPad Prism software.

### 4. Total RNA isolation

RNA extraction was performed using the Trizol LS (Invitrogen) method according to the manufacturer's protocol. In order to improve the yield and quality of the extracted RNA, time for cell lysis in Trizol LS was extended to 15 min, the first centrifugation step was extended to 20 min, and all centrifugation steps were performed at 16,000 rpm.

### 5. Real-Time PCR

Reverse Transcription

Each 2 µg of RNA were treated according to the manufacturer's protocol with DNAse (DNase I, 1000 U/µL, Roche Diagnostics, Basel, Switzerland). The whole sample of DNase treated RNA was then transcribed to cDNA using oligodT primers and SuperScript II Reverse Transcriptase Kit as recommended (Invitrogen).

Probe design

For Real-Time PCR, primers and MGB TaqMan probes, labeled at the 5'-end with the fluorescent dye FAM (6-carboxyfluorescein), were designed using Primer Express® Software and synthesized by Applied Biosystems (Foster City, CA, USA). All sequences for primers and probes can be found in Table 3.

**Table 3: Details of PAH-regulated subset genes, investigated by Real-Time PCR (primers and probe sequences)**

| **abbreviation** | **ID on microarray** | **forward primer** | **reverse primer** | **Taq Man Probe** |
|---|---|---|---|---|
| housekeeping gene | | | | |
| *gapdh* | | GGAGAAGGCCTCCATGCAT | TGGAGCCGAGATGACAACCT | TGAAGGGAGGTGCCAAG |

| target genes identified from microarray analyses: T-Test p<0.01, without multiple testing correction | | | | |
|---|---|---|---|---|
| *hsp20* | **003963* | CGAAAAGTGAGGATGAAGGAATCT | TTCCTGTCGAGGATGAATGAATT | CGTACACTCTCGTTTCGA |
| *proteas* | **002355* | GTGTAAGAAGGCGGTGAGTCATG | GTCCTAATAATACCCCCCGAACTT | CATGGCGAGGGATG |
| *dhg* | **007406* | AAGCCGTTGGAGCAGCATA | GCAGACTCTCGTTCAGGATTGG | AAGTTGAAGCTTGACGCAA |
| *eif2ak3* | **009962* | TGGAGGCACGGGAGGAT | CCAGCAGTCTTCGATAAGCATCT | TTGGCCTCATGAACTT |

| target genes identified from microarray analyses: T-Test p<0.05, with multiple testing correction (Benjamin-Hochberg) | | | | |
|---|---|---|---|---|
| *aatp* | *006218 | TGGCATCTCCGTTGTCTTTG | CGAACGAGATCAGGGAAGCA | CCTGATGGGAGGTACAG |
| *mcllhc* | *007357 | TGCCAAGGTTGATTCAAAAGG | TTAGATATCGTCTGCCCAGTCATC | ACGAAGTGGATGATTG |
| *tp8567 unknown* | *008567 | CTTCCTCGTCGGTCCTTCTAGA | CCAACCCACCGCGTTGT | CAAACAGACGAGCGGAC |

Real-Time PCR

Amplification reactions were performed with 12.5 µl of double concentrated Master Mix (Applied Biosystems), 0.9 mM each primer, 0.1 mM probe, and 2.5 µl of 1:15 diluted cDNA in a final volume of 25 µl. Samples were loaded in triplicate on 96-well optical reaction plates and PCR was performed in the ABI Prism 7900HT sequence detection system (Applied Biosystem). The manufacturer's recommended universal thermal cycler protocol was used: 2 min preheating at 50 ºC, 10 min at 95 ºC, followed by 40 cycles with 15 s at 95 ºC and 1 min at 60 ºC.

Gene expression data from Real-Time PCR were evaluated using Q-Gene (Muller et al, 2002), which takes into account the amplification efficiencies of target and reference genes. For all Real-Time PCR results, gapdh was used as housekeeping gene. gapdh threshold cycles for amplification in Real-Time PCR did not change significantly from each other for none of the different treatments (One way ANOVA). The calibrator sample was the control sample containing only the carrier solvent methanol (termed 0). For each exposure condition, the control plus four PAH treated samples were investigated. Samples were covering a range from "no effect" on growth up to a severe growth inhibition (≥ 40 %).

### 6. Probe and microarray design

60mer probes for spotting on the array were designed using the software Array Designer using the whole transcriptome available from JGI Thaps 3.0 database. For finding probes with appropriate design for the Agilent standard protocols, the Array Designer default values were applied, with the exception of the probe length with default 60mer probes (60 ± 0 nucleotides) and a melting temperature of 80 ± 5°C. Only in very exceptional cases where no probe could be identified under the default conditions, the range of the default values was widened; this was the case for < 1 % of the probes. Using these conditions, among the 11402 probes designed, 984 were rated with best quality, 9889 with good quality and 529 with poor quality.

The design comprised 4 probe groups: probe group 1 including 9709 probes for all transcript sequences where suitable probes were found and excluding housekeeping genes and positive controls. Probes from probe group 1 were all spotted 1 x on the array. Probe group 2 comprised six housekeeping genes, which were spotted each 3 times on the array. Probe group 3 contained probes for 11 selected positive controls, which were also spotted each 3 times. Probe group 4 contained 1680 genes for which cross homology could not be avoided in the probe design. Finally, a 5th probe group was added, which contained the 50 best rated probes from probe group 1-4 and which were spotted each 15 times on the array in order to be used as intra-array control.

This design so finally comprised 12190 spots. In addition, hybridization controls for artificial RNA mixed with the samples for quality control, are added on the array in the standard Agilent protocol. Since the standard Agilent customized array 8x 15K can comprise up to 15208 spots, the array design was automatically filled with probes from probe group 1 to the maximum possible. The array design was submitted to the company (Agilent), which manufactured the microarray using their standard on slide in situ synthesis technique. Probes are randomly distributed across the array.

### 7. Preparation of cRNA for hybridization to microarrays

1 µg of total RNA was used for transcription. Sample preparation was performed using the Agilent One color low RNA input linear Amplification Kit and the standard protocol. Thus, to the 1 µg of RNA sample, each 5 µL of diluted hybridization control (Agilent One Color RNA Spike-In Kit according to Agilent standard protocol) were added. Then total RNA was transcribed to cDNA, which was then in one step transcribed to cRNA and labeled with Cy3.

Yields of cRNA prepared from 1 µg of total RNA varied between 5 and 13 µg. Quality of total RNA and labeled cRNA were checked using the Bioanalyzer. Furthermore, labeling quality was checked on NanoDrop to assess the ratio of pmol dye/ng cRNA. Cy3-ratios ranged from 9.0 to 15.5. According to manufacturer's recommendation only samples with a ratio > 8 should be used.

### 8. Hybridization and scanning of microarray

600 ng of Cy3-labeled cRNA samples were hybridized on the customized microarrays according to the Agilent standard protocol and scanned on an Agilent scanner.

The hybridization controls showed always good quality, i.e. linearity for the different concentrations used in the spike-in kit. Data files were generated using Agilent Feature Extraction standard protocol.

### 9. Microarray data evaluation

Data from feature extraction were imported into Agilent Gene Spring 7 for data evaluation. They were normalized using the Gene Spring algorithm for 1 color microarrays. Then, flag filtering yielded only those signals, which were present or marginal in at least 3 out of 6 samples. A filtering on expression level followed to exclude all signals, which were regulated less than 2fold compared to the treatment. Among the remaining genes, those significant in a T-Test at a level of p < 0.05 and which passed the Multiple Testing Correction were picked (see Table 5).

Genes, which were highly and statistically significantly regulated on the microarray, were investigated further using real-time PCR.

**Table 5: Regulation of PAH-regulated subset genes on the microarray and in real-time PCR analysis**

| **abbreviation** | **SEQ ID NO** | **ID on microarray** | **Fold change respect to the control on microarray** | **Fold change respect to the control in real-time PCR** |
|---|---|---|---|---|
| | | | **average** | **average** |
| *hsp20* | 1 | *003963 | 95.5 (up-regulated) | 160 (up-regulated) |
| *proteas* | 2 | *002355 | 3.8 (up-regulated) | 6.16 (up-regulated) |
| *dhg* | 3 | *007406 | 0.02 (down-regulated) | 0.098 (down-regulated) |
| *eif2ak3* | 4 | *009962 | 0.06 (down-regulated) | 0.162 (down-regulated) |
| *aatp* | 5 | *006218 | 0.36 (down-regulated) | 0.28 (down-regulated) |
| *mcllhc* | 6 | *007357 | 0.57 (down-regulated) | 0.424 (down-regulated) |
| *tp8567 unknown* | 7 | *008567 | 0.7 (down-regulated) | 0.5 (down-regulated) |

## Claims

1. A nucleic acid selected from SEQ ID NO:1-7, or a fragment thereof.

2. A composition for detection of gene, said composition comprising at least one polynucleotide probe, wherein said at least one polynucleotide probe is selected from polynucleotide sequences SEQ ID NO:1-7.

3. The composition according to claim 2, wherein the composition comprises at least one polynucleotide probe of polynucleotide sequence SEQ ID NO:1 and at least one polynucleotide probe of polynucleotide sequences SEQ ID NO:2-7.

4. The composition according to claim 2, wherein the composition comprises one or more polynucleotide probes of polynucleotide sequences SEQ ID NO:1-4 and optionally further probes selected from SEQ ID NO:5-7

5. The composition according to any of claims 2 to 4 comprising at least one further polynucleotide probe of polynucleotide sequence SEQ ID NO:8 and/or of polynucleotide sequence SEQ ID NO:9.

6. The composition according to any of claims 2 to 5 further comprising a polynucleotide probe of polynucleotide sequence SEQ ID NO:10 as a control.

7. The composition according to any of the previous claims, wherein the composition is a microarray comprising a support on which said at least one polynucleotide probes has/have been immobilized.

8. Use of a nucleic acid according to claim 1 as an hybridization probe, preferably immobilized on a microarray.

9. A method of detecting polycyclic aromatic hydrocarbon pollutants in water comprising the steps:
(a) culturing Thalassiosira pseudonana cells in said water,
(b) isolating nuleic acid from said culture,
(c) detecting level of gene expression using a composition according to any of claims 2-7.

10. A method of assessing toxicity of a polycyclic aromatic hydrocarbon test compound comprising the steps:
(a) culturing Thalassiosira pseudonana cells in a medium comprising varying concentrations of said test compound,
(b) obtaining nuleic acid from said culture by isolation,
(c) detecting level of gene expression using a composition according to any of claims 2-7.


SEQUENCE LISTING

<110> EUROPEAN COMMISSION

<120> ECOTOXICOLOGICAL RESPONSE INDICATORS

<130> P-2817/EP

<160> 10

<170> PatentIn version 3.5

<210> 1
<211> 60
<212> DNA
<213> Thalassiosira pseudonana

<400> 1
tgacaatcgc ttgttgagga ttactggtga gacgaaaagt gaggatgaag gaatctccgt 60

<210> 2
<211> 60
<212> DNA
<213> Thalassiosira pseudonana

<400> 2
ggggtattat taggactgtg gtggcgacgg aggaggggaa tgctagggat catgttttgg 60

<210> 3
<211> 60
<212> DNA
<213> Thalassiosira pseudonana

<400> 3
taccagacta tcagttcccc gttggggact cagctcactc aagccgttgg agcagcatac 60

<210> 4
<211> 60
<212> DNA
<213> Thalassiosira pseudonana

<400> 4
agatgcttat cgaagactgc tggagccacg atatgaggca acggccagaa atgaaaggtt 60

<210> 5
<211> 60
<212> DNA
<213> Thalassiosira pseudonana

<400> 5
agctgcttcc ctgatctcgt tcgtccttcc tggattgttt ctgcttcaga ctggagatgg 60

<210> 6
<211> 60
<212> DNA
<213> Thalassiosira pseudonana

<400> 6
aaaggggacg aagtggatga ttggggtgat gactgggcag acgatatcta atgcaatgca 60

<210> 7
<211> 60

<212>  DNA
<213>  Thalassiosira pseudonana

<400>  7
agtgcccaaa ctaggtacag aatttgacgg gaagcaacta ggaccctggt tctacgctcc      60



<210>  8
<211>  60
<212>  DNA
<213>  Thalassiosira pseudonana

<400>  8
catgggcacg ggacaatcta cccgatctcg ccactgcctc gttcttagat ctgtgcaacc     60



<210>  9
<211>  60
<212>  DNA
<213>  Thalassiosira pseudonana

<400>  9
gtgtgtggaa  ctacgtgctg ccttcgcttc ccaggtcagc tcaactgcga tctccgaaag  60



<210>  10
<211>  60
<212>  DNA
<213>  Thalassiosira pseudonana

<400>  10
cgtctccaac gcctcttgta ccaccaactg tcttgctcct cttgccaagg ttgtcaacga   60

SEQUENCE LISTING

<110>  EUROPEAN COMMISSION

<120>  ECOTOXICOLOGICAL RESPONSE INDICATORS

<130>  P-2817/EP

<160>  10

<170>  PatentIn version 3.5

<210>  1
<211>  60
<212>  DNA
<213>  Thalassiosira pseudonana

<400>  1
tgacaatcgc ttgttgagga ttactggtga gacgaaaagt gaggatgaag gaatctccgt        60


<210>  2
<211>  60
<212>  DNA
<213>  Thalassiosira pseudonana

<400>  2
ggggtattat taggactgtg gtggcgacgg aggaggggaa tgctagggat catgttttgg        60


<210>  3
<211>  60
<212>  DNA
<213>  Thalassiosira pseudonana

<400>  3
taccagacta tcagttcccc gttggggact cagctcactc aagccgttgg agcagcatac        60


<210>  4
<211>  60
<212>  DNA
<213>  Thalassiosira pseudonana

<400>  4
agatgcttat cgaagactgc tggagccacg atatgaggca acggccagaa atgaaaggtt        60


<210>  5
<211>  60
<212>  DNA
<213>  Thalassiosira pseudonana

<400>  5
agctgcttcc ctgatctcgt tcgtccttcc tggattgttt ctgcttcaga ctggagatgg        60


<210>  6
<211>  60
<212>  DNA
<213>  Thalassiosira pseudonana

<400>  6
aaaggggacg aagtggatga ttggggtgat gactgggcag acgatatcta atgcaatgca        60


<210>  7
<211>  60
<212>  DNA
<213>  Thalassiosira pseudonana

<400>  7

agtgcccaaa ctaggtacag aatttgacgg gaagcaacta ggaccctggt tctacgctcc 60

<210> 8
<211> 60
<212> DNA
<213> Thalassiosira pseudonana

<400> 8
catgggcacg ggacaatcta cccgatctcg ccactgcctc gttcttagat ctgtgcaacc 60

<210> 9
<211> 60
<212> DNA
<213> Thalassiosira pseudonana

<400> 9
gtgtgtggaa ctacgtgctg ccttcgcttc ccaggtcagc tcaactgcga tctccgaaag 60

<210> 10
<211> 60
<212> DNA
<213> Thalassiosira pseudonana

<400> 10
cgtctccaac gcctcttgta ccaccaactg tcttgctcct cttgccaagg ttgtcaacga 60